# EUROPÄISCHE PATENTANMELDUNG

(11) **EP 3 254 679 A1**
(43) Veröffentlichungstag der Anmeldung: **13.12.2017**
(21) Anmeldenummer: 16173828.1
(22) Anmeldetag: 09.06.2016
(51) Int. Cl.: A61K 31/035, A61P 31/00, A61P 31/04

(54) **WIRKSTOFFE GEGEN PROTOZOEN UND BAKTERIEN**

(71) Anmelder: Christian-Albrechts-Universität zu Kiel, 24118 Kiel (DE)
(72) Erfinder: Beitz, Eric, 24229 Dänischenhagen (DE); Golldack, André, 24143 Kiel (DE); Henke, Björn, 24116 Kiel (DE)
(74) Vertreter: Taruttis, Stefan Georg

(57) **Zusammenfassung**

Die Erfindung betrifft Verbindungen, die gegen Protozoen und/oder Bakterien gerichtet sind, zur Verwendung bei der Behandlung von Infektionen durch Protozoen und/oder Bakterien. Die Verbindungen zeichnen sich dadurch aus, dass sie den Formiat-Nitrit-Transporter von Protozoen und/oder Bakterien hemmen.

## Beschreibung

Die vorliegende Erfindung betrifft Verbindungen, die gegen Protozoen und/oder Bakterien gerichtet sind und insbesondere die Verbindungen zur Verwendung als Arzneimittel, bevorzugt bei der Behandlung von Infektionen durch Protozoen und/oder Bakterien. Des Weiteren betrifft die Erfindung ein Verfahren zur Hemmung, bevorzugt Zerstörung, von Protozoen und/oder Bakterien durch Kontaktieren von Protozoen und/oder Bakterien mit zumindest einer der Verbindungen. Die Verbindungen zeichnen sich dadurch aus, dass sie den Formiat-Nitrit-Transporter von Protozoen und/oder Bakterien hemmen.

### Stand der Technik

Die EP 2 483 274 B1 beschreibt eine Vielzahl von Wirkstoffen gegen Malaria, die die Dihydroorotatdehydrogenase von Plasmodium hemmen sollen.

Die EP 2 526 090 B1 beschreibt Aminopyridinderivate als pharmazeutischen Wirkstoff insbesondere gegen Malaria.

Die Organisation Medicines for Malaria Venture (MMV) beschreibt als Wirkstoffe gegen Malaria unter insgesamt ca. 400 anderen Verbindungen die folgenden:

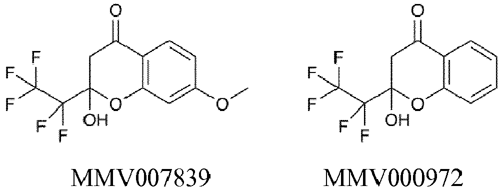

### Aufgabe der Erfindung

Die Aufgabe der Erfindung liegt in der Bereitstellung von alternativen Verbindungen, die als Wirkstoffe insbesondere gegen Protozoen und/oder Bakterien wirksam sind. Bevorzugt sind die alternativen Verbindungen gegen ein anderes Zielmolekül von Protozoen und/oder Bakterien wirksam als es bislang bekannt ist.

### Beschreibung der Erfindung

Die Erfindung löst die Aufgabe mit den Merkmalen der Ansprüche, insbesondere mittels einer Verbindung und deren Derivaten zur Verwendung als Arzneimittel, insbesondere gegen Protozoen und/oder Bakterien, wobei die Verbindung die folgende Struktur I aufweist oder daraus besteht, in der der Rest R1 ein Hyperfluoralkyl ist, bei dem das Alkyl ein gradkettiges oder verzweigtes C₁- bis C₄-alkyl ist: 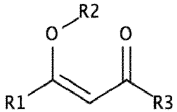 Struktur I, mit
R1 = Hyperfluor-C₁- bis C₄-alkyl, gradkettig oder verzweigt, insbesondere Trifluormethyl, Pentafluorethyl, Heptafluorpropyl, davon bevorzugt Pentafluorethyl, einschließlich Solvaten und Salzen dieser.
R2 ist H oder ein C₁- bis C₁₂-Alkyl, bevorzugt H oder Ethyl. Auf Grundlage der derzeitigen Analysen ist anzunehmen, dass das wirksame Pharmakophor von Struktur I mit R1 gebildet wird.
R3 ist bevorzugt ein Aromat, bevorzugt ein 5-Ring oder ein 6-Ring, der optional daran kondensierte aromatische Ringe aufweisen kann. Der Aromat kann direkt oder über ein C₁-oder C₂-Alkyl gebunden sein. Bevorzugt weist R3 eine Hydroxylgruppe am zweiten Kohlenstoffatom auf, entsprechend der ortho-Stellung, wenn R3 ein aromatischer 6-Ring ist. Denn eine Hydroxylgruppe in R3 im Abstand von zwei Kohlenstoffen zum Carbonyl-Kohlenstoff der Struktur I führt zur Ketalbildung; das Ketal stellt ein gut membrangängiges internes Prodrug dar. Z.B. ist R3 aus der Gruppe ausgewählt, die einkernige Aromaten, die in ortho-Stellung zur Struktur I eine Hydroxylgruppe aufweisen und/oder in para-Stellung zur Struktur I mit einem gradkettigen oder verzweigten C₁- bis C₁₂-Alkylrest, C₁- bis C₁₂-Alkyloxyrest oder Halogen substituiert sind, insbesondere die folgenden Reste 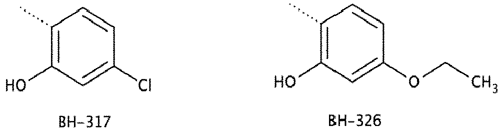 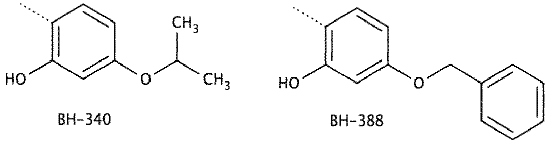 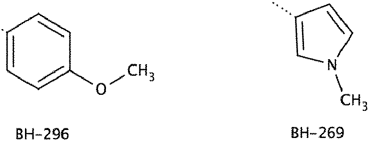 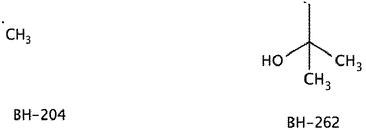 umfasst oder daraus besteht. Die Bezeichnungen unter den Resten R3 nennen die Namen der Verbindungen aus Struktur I mit R1 = Hyperfluor-C₁- bis C₄-alkyl, bevorzugt Pentafluorethyl, R2 = H oder Ethyl, bevorzugt H und dem angegeben Rest für R3.

Der einkernige Aromat kann ein Phenyl, ein Pyrrolyl, Furyl oder Pyridyl sein.

Erfindungsgemäß ist die hydrolysierte, offenkettige vinyloge Carbonsäure die aktive, substratähnliche Wirkform, während die Ketale reversible, interne Prodrugs mit guter Resorption in Malaria-Parasiten darstellen. Die Nutzung des Prodrug-Prinzip durch Einführung einer Hydroxylgruppe in R3 kann somit die Verfügbarkeit im Parasiten erhöhen.

Diese Verbindungen zeichnen sich dadurch aus, dass sie das Formiat-Nitrit-Transporterprotein (FNT) aus Bakterien und Protozoen hemmen. Der Mensch besitzt diese Formiat-Nitrit-Transporterproteine nicht. Der humane Laktat-Transporter wird durch die Verbindungen wesentlich schlechter gehemmt. Die Hemmung des FNT führt dazu, dass in den Bakterien Formiat und Nitrit bzw. in den Protozoen Acetat und Laktat, das z.B. bei der anaeroben Verstoffwechselung von Glucose aus Pyruvat entsteht, nicht effektiv transportiert wird, so dass die Zellen z.B. übersäuern und absterben.

Generell weisen nur Bakterien und Protozoen, gegen die die Verbindungen als Wirkstoff verwendet werden, Formiat-Nitrit-Transporterproteine (FNT) auf. Zur Anwendung der Verbindungen bevorzugte Bakterien sind Gram-negativ, z.B. Salmonella thyphimurium (bevorzugt enthaltend das FNT St; StNirC, Sequence-Accession-No. AAA27040 NCBI, bevorzugt SEQ ID NO: 6). Zur Anwendung der Verbindungen bevorzugte Protozoen sind einzellige Parasiten von Mensch und Tier, insbesondere die folgenden, die das angegebene FNT aufweisen: Plasmodium falciparum (Pf; PfFNT, FNT: Sequence-ID PF3D7_0316600 PlasmoDB, bevorzugt SEQ ID NO: 1), der Erreger der Malaria, Toxoplasma gondii (Tg; FNT: TgFNT1, Sequence-ID TGGT1_209800 ToxoDB, bevorzugt SEQ ID NO: 2; TgFNT2, FNT: Sequence-ID TGGT1_292110 ToxoDB, bevorzugt SEQ ID NO: 3; TgFNT3, FNT: Sequence-ID TGGT1_229170 ToxoDB, bevorzugt SEQ ID NO: 4), der Erreger der Toxoplasmose, Entamoeba histolytica (Eh; EhFNT, Sequence-ID FNT: EHI_198990 AmoebaDB, bevorzugt SEQ ID NO: 5), der Erreger der Amöbenruhr.

Die Erfindung wird nun genauer anhand von Beispielen erläutert.

### Beispiel 1: Wirkung von Verbindungen gegen FNT

Die Wirkung der folgenden Verbindungen auf das Wachstum von Plasmodium falciparum wurde anhand kultivierter Plasmodien getestet:

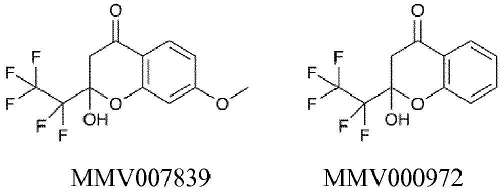

Die Hemmwirkung IC50 auf das Wachstum der Plasmodiumkultur wurde für MMV007839 zu 140 nM bestimmt, für MMV000972 zu 1,7 µM. Die Plasmodiumkultur wurde in 5% 0+ Erythrocyten in RPMI 1640 Medium mit 0,5% Albumax bei 37 °C geführt. Die IC50 wurde 48 h nach Zugabe der Substanz-Verdünnungen über Auszählung der Parasitämie per FACS bestimmt. Nach 24 h erfolgte ein Mediumwechsel und die Verbindungen wurden frisch zugegeben.

Bei der Behandlung der kultivierten Plasmodien mit MMV007839 bei einer Konzentration entsprechend der dreifachen IC50-Konzentration wurden resistente Plasmodien erhalten, deren IC50 sich zu 35 µM verschoben hatte. Eine Sequenzierung des FNT-Gens der resistenten Plasmodien ergab eine Mutation Gly107Ser auf Proteinebene.

Mit Ausnahme der Verbindung BH-296 ist bislang keine Verbindung bekannt, die im pharmazeutisch akzeptablen Konzentrationsbereich auch gegen die in Beispiel 1 beschriebenen resistenten Parasiten wirksam ist.

Der FNT des Plasmodium-Wildtyps (erhältlich unter Zugangsnr. PF3D7_0316600 bei der Datenbank PlasmoDB) und der resistenten Plasmodien (Position der Mutation im offenen Leserahmen: Guanin319 zu Adenin) wurden in der Hefe Saccharomyces cerevisiae W303-1A jen1Δ ady2Δ vom Plasmid pDR196 (PMA Promotor) exprimiert. In der Hefe waren die endogenen Gene für die Monocarboxylat-Transporter Jenlp (Sequence-Accession-No. CAA82062 NCBI) und Ady2p (Sequence-Accession-No. KZV12856) deletiert (bezogen von M. Casal Universidade do Minho, Portugal).

Diese Hefe wurde in SD Medium unter Zusatz von Adenin, Histidin, Leucin, Tryptophan und 2% (wt/V) Glucose bei 30°C bis zu einer OD₆₀₀ von 0,8 bis 1,0 wachsen gelassen, durch Zentrifugieren geerntet und einmal mit Wasser gewaschen und erneut abzentrifugiert und in 50 mM HEPES/TRIS (pH 6,8) suspendiert und auf eine OD₆₀₀ von 50 ±10 % eingestellt und auf Eis gelagert. Zur Kontaktierung mit einer der Verbindungen wurde eine Verbindung einer Stufe einer Verdünnungsreihe in DMSO in einem Reaktionsgefäß vorgelegt und darauf jeweils 80 µl der Hefesuspension pipettiert. Nach einer Inkubation auf Eis für 15 bis 20 min werden 20 µl 5 mM Na-L-Laktat plus 0,04 µCi radioaktiv markiertes L-[1-¹⁴C]-Laktat zugegeben. Die erhaltene Laktat-Konzentration betrug 1 mM. Nach einer Inkubation von 30 s wird die Aufnahme von Laktat durch schnelles Verdünnen mittels Zugabe von 1 ml eiskaltem Wasser gestoppt. Aus der so verdünnten Hefesuspension wurden die Hefezellen durch Vakuumfiltration auf eine Filtermembran gebracht, mit 7 ml kaltem Wasser gewaschen und mit der Filtermembran in 3 ml Szintillationscocktail überführt. Nach 24 h Inkubation bei 18°C in dem Szintillationscocktail, in dem die Zellen lysiert wurden, wird die Menge radioaktiv markierten Laktats mittels eines Szintillationszählers gemessen. Aus der gemessenen Menge radioaktiv markierten Laktats wurde die von der Hefe aufgenommene Gesamtmenge Laktat berechnet. Als Positivkontrolle wurde in Parallelansätzen die Hefe mit DMSO ohne eine der Verbindungen mitgeführt und als 100 % FNT-Aktivität bzw. 0 % Hemmung angesehen. In Ansätzen mit Verbindung wurde eine im Vergleich mit den parallelen Positivkontrollen geringere Menge aufgenommenen Laktats gemessen. Als Negativkontrolle wurde Hefe mitgeführt, in der die endogenen Gene für die Monocarboxylat-Transporter Jenlp und Ady2p deletiert waren, jedoch nicht die FNT aus Plasmodium exprimiert wurde. Diese Negativkontrolle wurde als 0 % FNT-Aktivität bzw.100 % Hemmung angesehen.

Die mit der Hefe bestimmten IC50-Werte für das Wildtyp-FNT und die FNT-Mutante zeigten dieselbe Verschiebung wie die IC50-Werte der Plasmodium-Kultur. Dies zeigt zum einen, dass die getesteten Verbindungen ihre Wirkung auf Plasmodium durch Wechselwirkung mit dem FNT entfalten, bzw. dass FNT das Zielmolekül dieser Verbindungen bei der Hemmung von Plasmodium ist. Zum anderen zeigt dieses Ergebnis, dass die den FNT aus Plasmodium exprimierende Hefe stellvertretend für Plasmodium selbst in Verfahren zur Analyse der Hemmwirkung von Verbindungen auf Plasmodium eingesetzt werden kann. Die Ausbildung der Ketal-Prodrugform wirkt sich generell positiv auf die Wirksamkeit in Plasmodien-Kultur aus, während die Hefe die offenkettige Form ebenso gut aufnimmt.

### Beispiel 2: Herstellung von Verbindungen

Die Herstellung des Grundkörpers der Verbindungen kann durch Synthese mit den folgenden Schritten erfolgen: 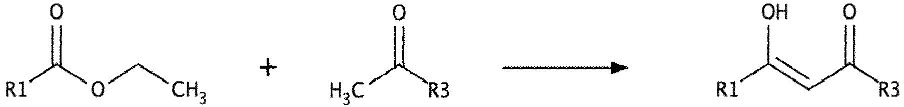 mit R1 = Hyperfluor-C₁- bis C₄-alkyl,
R3 einer von 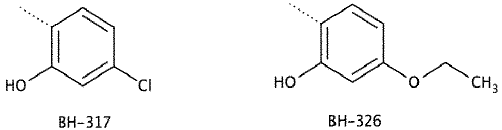 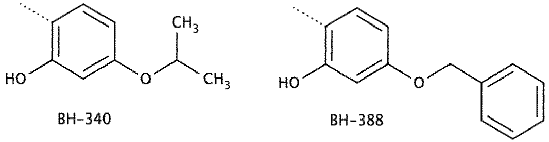 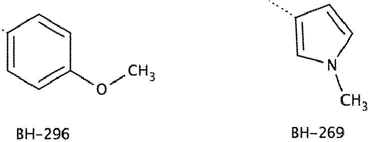 CH₃ BH-204

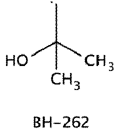

Für die Synthese werden 7,5 ml wasserfreies Tetrahydrofuran (THF) und 0,34 g (42,8 mmol) fein dispergiertes Lithiumhydrid in einem getrockneten 100 ml-Dreihalskolben vorgelegt, mit einem Rückflusskühler und einem Tropftrichter versehen, gerührt und zum Sieden erhitzt. Zu dieser Suspension wird ein Gemisch aus 15,0 mmol Hyperfluoralkylcarbonsäure-Ethylester, dessen Hyperfluoralkylgruppe R1 entspricht, und 12,5 mmol einer 2-Ketoverbindung entsprechend R3, gelöst in getrocknetem THF, langsam zugetropft. Das Reaktionsgemisch wird 3 h unter Rückfluss gekocht. Anschließend wird das THF am Rotationsverdampfer entfernt und der erhaltene Rückstand wird mit 60 ml einer kalten Mischung aus Essigsäure und Wasser (7:50) versetzt und das Gemisch mit 2 x 100 ml Ethylacetat extrahiert. Die vereinigten Phase werden über Natriumsulfat getrocknet, einrotiert, über Kieselgel säulenchromatographisch aufgereinigt (Cyclohexan/Ethylacetat 90:10) und in n-Hexan umkristallisiert. Die Struktur der Verbindungen wird mittels 1H-NMR, 13C-NMR und Massenspektrometrie (LC-MS ESI) bestätigt. Die Veresterung der erhaltenen vinylogen Carbonsäure gemäß 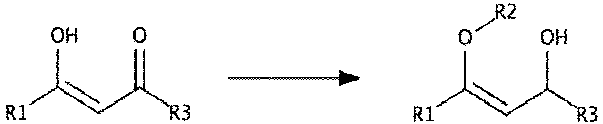 erfolgte durch Vorlegen von 5,0 ml wasserfreiem Dimethylformamid (DMF) mit 2 mmol der vinylogen Carbonsäureverbindung und 2 mmol (652 mg) Cäsiumcarbonat in einem getrockneten 100 ml-Dreihalskolben, mit Rückflusskühler und Tropftrichter versehen, und Rühren für 1 h bei 70 °C. Zu der Suspension werden 2,2 mmol (442 mg) p-Toluolsulfonsäureethylester, gelöst in 5 ml getrocknetem DMF, langsam zugetropft und 6 h bei 70 °C gerührt. Das Gemisch wird auf Wasser gegeben, 2 x mit Diethylether extrahiert und die organischen Phasen über Natriumsulfat getrocknet.

Nach dem Einengen des Ethers am Rotationsverdampfer wird das erhaltene Rohprodukt über Kieselgel säulenchromatographisch aufgereinigt (Cyclohexan/Ethylacetat/Diethylamin 94:5:1). Die Struktur der Verbindungen wird mittels 1H-NMR, 13C-NMR und Massenspektrometrie (LC-MS ESI) bestätigt.

### Beispiel 3: Wirkung von Verbindungen gegen FNT

Stellvertretend für ein Formiat-Nitrit-Transporterprotein (FNT) aus Bakterien oder Protozoen wurde das FNT von Plasmodium falciparum als Wildtyp und als Gly107Ser-Mutante verwendet, die jeweils in Hefe exprimiert und mit Verdünnungen von zu testenden Verbindungen inkubiert wurden, wie in Beispiel 1 beschrieben.

Dabei wurden die Verbindungen 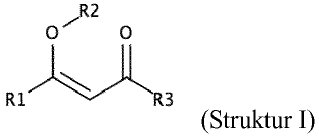 mit R1 = Pentafluorethyl oder Heptafluorpropyl (BH-340), R2 = H und
R3 einer der Reste 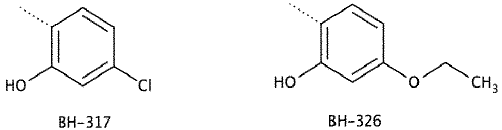 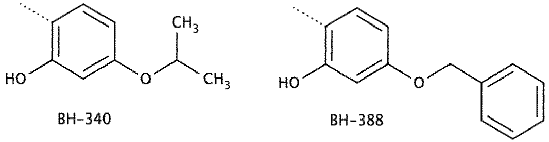 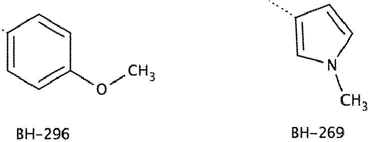 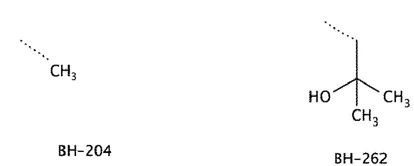 eingesetzt, die jeweils nach der Bezeichnung benannt wurden, der unter R3 angegeben ist.

Die folgenden Hemmwerte wurden erhalten:

| | Hefe-Testsystem | | Plasmodium-Kultur | |
|---|---|---|---|---|
| Verbindung | IC50 gegen FNT Wildtyp (µM) | IC50 gegen Gly107Ser-Mutante von FNT (µM) | IC50 gegen Wildtyp (µM) | IC50 gegen Gly107Ser-Mutante (µM) |
| BH-317 | 0,12 | nicht bestimmt | 0,28 | nicht bestimmt |
| BH-326 | 0,24 | nicht bestimmt | 0,05 | nicht bestimmt |
| BH-340 | 0,40 | nicht bestimmt | 0,10 | nicht bestimmt |
| BH-388 | 0,88 | nicht bestimmt | nicht bestimmt | nicht bestimmt |
| BH-296 | 0,14 | 2,3 | 3,6 | 8,6 |
| BH-269 | 0,12 | 14,0 | nicht bestimmt | nicht bestimmt |
| BH-204 | 1,9 | 8,1 | nicht bestimmt | nicht bestimmt |
| BH-262 | 2,0 | nicht bestimmt | 10,4 | 70,4 |
| BH-362 | 0,25 | nicht bestimmt | nicht bestimmt | nicht bestimmt |

Die IC50-Werte zeigen, dass die getesteten neuen Verbindungen den FNT von Plasmodium signifikant hemmen.

Weiterhin zeigen die IC50-Werte, dass die Verbindungen BH-269 und bevorzugt BH-296 auch die Gly107Ser-Mutante signifikant hemmen.

Die Verbindungen zeigen eine Abhängigkeit der IC50 von der Zeit der Vorinkubation, bevor Laktat zu den Zellen gegeben wird. Dabei zeigt sich, dass eine längere Vorinkubation zu einer erhöhten Transporthemmung führt. Bei Verlängerung der Vorinkubation auf 24 h vor der Zugabe von Laktat wird ein IC50 von 15 nM anstelle des IC50 von 170 nM nach 20 min bestimmt. Die Hemmung des FNT konnte nicht durch Waschen der Zellen mit 50 mM HEPES-Tris, pH6,8 verringert werden und die Hemmung blieb über Stunden erhalten. Daher wird angenommen, dass die Verbindung jedenfalls durch Waschen nicht vom FNT entfernt wird. Dieses Ergebnis deutet daraufhin, dass die Verbindungen irreversibel an den FNT binden, bzw. die Verbindungen eine Dauerblockade des FNT durch Suizidhemmung verursachen. Es wird vermutet, dass die Verbindungen die Struktur zweier Laktat-Moleküle nachahmen, eines in der anionischen Form (vinyloge Carbonsäure der Struktur I) und eines in der neutralen Milchsäureform (Fluoralkyl-Rest, R1).

Daher wird gegenwärtig angenommen, dass das Pharmakophor von Struktur I mit dem Hyperfluor-C₁- bis C₄-alkyl als R1 gebildet wird und der Rest R3 variiert werden kann, z.B. sterisch deutlich über einen einkernigen aromatischen Rest R3 erweitert werden kann. Das Fluoralkyl R1 kann in den lipophilen Transportkanal des FNT ragen, während die negativ geladene Struktur I elektrostatisch mit dem Transportereingang des FNT interagiert.

## Patentansprüche

1. Verbindung zur Verwendung als Arzneimittel, die
die Struktur I aufweist, in der der Rest R1 ein Hyperfluoralkyl ist, bei dem das Alkyl ein gradkettiges oder verzweigtes C₁- bis C₄-alkyl ist

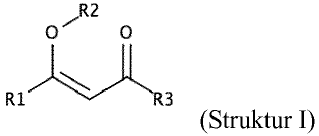

2. Verbindung nach Anspruch 1, **dadurch gekennzeichnet, dass** die Protozoen und/oder Bakterien ein Formiat-Nitrit-Transporterprotein (FNT) aufweisen.

3. Verbindung nach einem der voranstehenden Ansprüche, **dadurch gekennzeichnet, dass** die Verwendung als Arzneimittel zur Behandlung einer Infektion eines Menschen oder Tiers mit Protozoen und/oder Bakterien ist.

4. Verbindung nach einem der voranstehenden Ansprüche, **dadurch gekennzeichnet, dass** R2 H oder ein C₁- bis C₁₂-Alkyl ist.

5. Verbindung nach einem der voranstehenden Ansprüche, **dadurch gekennzeichnet, dass** R3 ein Aromat ist, der direkt oder über ein C₁- oder C₂-Alkyl gebunden ist, und der in para-Stellung zur Struktur I mit einem gradkettigen oder verzweigten C₁- bis C₁₂-Alkylrest, C₁- bis C₁₂-Alkyloxyrest oder Halogen substituiert sind.

6. Verbindung nach einem der voranstehenden Ansprüche, **dadurch gekennzeichnet, dass** R3 eine Hydroxylgruppe im Abstand von zwei Kohlenstoffen zum Carbonyl-C-Atom der Struktur I zur Ausbildung des Ketal-Prodrugs enthält.

7. Verbindung nach einem der voranstehenden Ansprüche, **dadurch gekennzeichnet, dass** R3 aus der Gruppe ausgewählt ist, die einkernige Aromaten umfasst, die in para-Stellung zur Struktur I mit einem gradkettigen oder verzweigten C₁- bis C₁₂-Alkylrest substituiert sind.

8. Verbindung nach Anspruch 7, **dadurch gekennzeichnet, dass** R3 in ortho-Stellung zur Struktur I eine Hydroxylgruppe aufweist.

9. Verbindung nach einem der voranstehenden Ansprüche, **dadurch gekennzeichnet, dass**
R3 unter 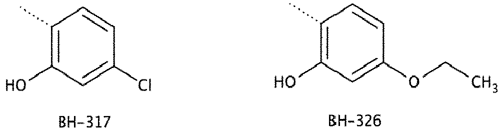 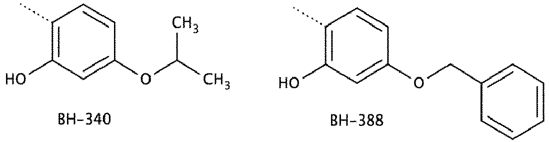 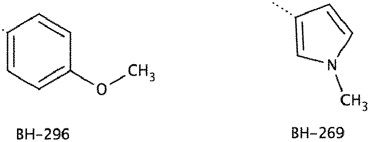 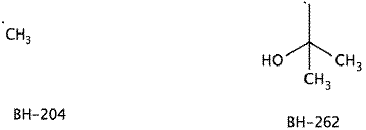 ausgewählt ist.

10. Verbindung nach einem der voranstehenden Ansprüche, **dadurch gekennzeichnet, dass** das Arzneimittel gegen Protozoen und/oder Bakterien gerichtet ist, die ein Formiat-Nitrit-Transporterprotein aufweisen.


SEQUENCE LISTING

<110> Christian-Albrechts-UniversitÃ¤t zu Kiel

<120> Wirkstoffe gegen Protozoen und Bakterien

<130> K1027EP

<160> 6

<170> BiSSAP 1.3

<210> 1
<211> 309
<212> PRT
<213> Plasmodium falciparum

<220>
<223> PfFNT_PF3D7_0316600_PlasmoDB

<400> 1
Met Pro Pro Asn Asn Ser Lys Tyr Val Leu Asp Pro Val Ser Ile Lys
1               5                   10                  15
Ser Val Cys Gly Gly Glu Glu Ser Tyr Ile Arg Cys Val Glu Tyr Gly
            20                  25                  30
Lys Lys Lys Ala His Tyr Ser Asn Leu Asn Leu Leu Ala Lys Ala Ile
        35                  40                  45
Leu Ala Gly Met Phe Val Gly Leu Cys Ala His Ala Ser Gly Ile Ala
    50                  55                  60
Gly Gly Leu Phe Tyr Tyr His Lys Leu Arg Glu Ile Val Gly Ala Ser
65                  70                  75                  80
Met Ser Val Phe Val Tyr Gly Phe Thr Phe Pro Ile Ala Phe Met Cys
                85                  90                  95
Ile Ile Cys Thr Gly Ser Asp Leu Phe Thr Gly Asn Thr Leu Ala Val
            100                 105                 110
Thr Met Ala Leu Tyr Glu Lys Lys Val Lys Leu Leu Asp Tyr Leu Arg
        115                 120                 125
Val Met Thr Ile Ser Leu Phe Gly Asn Tyr Val Gly Ala Val Ser Phe
    130                 135                 140
Ala Phe Phe Val Ser Tyr Leu Ser Gly Ala Phe Thr Asn Val His Ala
145                 150                 155                 160
Val Glu Lys Asn His Phe Phe Gln Phe Leu Asn Asp Ile Ala Glu Lys
                165                 170                 175
Lys Val His His Thr Phe Val Glu Cys Val Ser Leu Ala Val Gly Cys
            180                 185                 190
Asn Ile Phe Val Cys Leu Ala Val Tyr Phe Val Leu Thr Leu Lys Asp
        195                 200                 205
Gly Ala Gly Tyr Val Phe Ser Val Phe Phe Ala Val Tyr Ala Phe Ala
    210                 215                 220
Ile Ala Gly Tyr Glu His Ile Ile Ala Asn Ile Tyr Thr Leu Asn Ile
225                 230                 235                 240
Ala Leu Met Val Asn Thr Lys Ile Thr Val Tyr Gln Ala Tyr Ile Lys
                245                 250                 255
Asn Leu Leu Pro Thr Leu Leu Gly Asn Tyr Ile Ala Gly Ala Ile Val
            260                 265                 270
Leu Gly Leu Pro Leu Tyr Phe Ile Tyr Lys Glu His Tyr Tyr Asn Phe
        275                 280                 285
Glu Arg Ser Lys Arg Asp Asn Asn Asp Ala Gln Met Lys Ser Leu Ser
    290                 295                 300
Ile Glu Leu Arg Asn
305

<210> 2

<211> 412
<212> PRT
<213> Toxoplasma gondii

<220>
<223> TgFNT1_TGGT1_209800_ToxoDB

<400> 2
Met Val Val Thr Ala Ser Pro Asp Thr Tyr Leu His Val Ile Asp Tyr
1               5                   10                  15
Gly Leu Lys Lys Val Arg Leu Arg Phe Asp Arg Leu Leu Leu Gln Ala
            20                  25                  30
Phe Met Ala Gly Val Tyr Ile Gly Met Ala Gly Asn Ala Cys Ile Ser
        35                  40                  45
Leu Ala Gly Gly Phe Ser Thr Asp Pro Ala Asp Pro Lys Ala Ile Thr
    50                  55                  60
Ala Gly Val Gln Lys Phe Ile Tyr Ala Ser Ile Phe Pro Val Ala Phe
65                  70                  75                  80
Ile Ala Ile Ile Met Thr Gly Ala Glu Leu Phe Thr Gly Asn Thr Met
                85                  90                  95
Thr Met Leu Ile Cys Trp Phe Glu Arg Arg Ile Thr Ile Trp Gln Leu
            100                 105                 110
Leu Gln Asn Trp Ala Gly Ser Phe Leu Gly Asn Trp Leu Gly Thr Met
        115                 120                 125
Phe Ser Ala Tyr Phe Leu Thr Tyr Leu Cys Cys Pro Phe Asp His Asp
    130                 135                 140
Pro Tyr Leu Ser Tyr Leu Asn Tyr Thr Ala Ala Ser Lys Val Ser Tyr
145                 150                 155                 160
Gly Trp Gly Ser Cys Phe Leu Arg Gly Val Gly Cys Asn Thr Trp Val
                165                 170                 175
Cys Leu Ala Val Trp Phe Val Val Ala Cys Asp Asp Ala Ala Gly Lys
            180                 185                 190
Ile Leu Ala Leu Trp Phe Pro Ile Val Ala Phe Val Leu Ser Ser Tyr
        195                 200                 205
Glu His Ile Ile Ala Asn Leu Tyr Thr Leu Gln Leu Cys Ala Met Leu
    210                 215                 220
Gly Val Asp Thr Ser Leu Ala Asp Met Ile Ala Phe Asn Leu Leu Pro
225                 230                 235                 240
Thr Leu Leu Gly Asn Leu Phe Gly Gly Cys Gly Leu Ile Gly Met Val
                245                 250                 255
Tyr Phe Tyr Asn Phe Tyr Pro Val Val Gly His Gly Asp Asp Ala Ala
            260                 265                 270
Glu Gly Ser Ile Cys Gly Ser Ser Glu Lys Glu Glu Cys Pro Ser Leu
        275                 280                 285
Val Gly Val Pro Arg Gly Ala Ser Val Asn Ser Leu Ala Val Ser Ala
    290                 295                 300
Ile Pro Ser Val Phe Ser Ala Pro Arg Gly Gln Arg Glu Ser Phe Ala
305                 310                 315                 320
Gly Glu Ser Ser Thr Leu Val Met Gly Asp Ile Lys Arg Gln Arg Ser
                325                 330                 335
Met Ala Ser Thr Arg Lys Leu Gly Gly Gly Ala Asp Lys Lys Asp Val
            340                 345                 350
Gln Leu Thr Val Arg Gln Phe Asp Glu Thr Glu Met Gln Ser Thr Met
        355                 360                 365
Glu Asp Met Phe Gly Leu Glu Asp Pro Arg Asn Ala Pro Lys Gly Asn
    370                 375                 380
Pro Gly Thr Asn Pro Pro Ser Lys Ser Pro Glu Ser Ser Ala Thr Gly
385                 390                 395                 400
Gly Thr Gly Ala Ala Ala Ser Pro Thr Ala Thr Ser
                405                 410

<210> 3
<211> 463

<212> PRT
<213> Toxoplasma gondii

<220>
<223> TgFNT3_TGGT1_292110_ToxoDB

<400> 3
Met Cys Ser Ile Pro Pro Leu Arg Leu Leu Glu Val Val Asp Asp Leu
1               5                   10                  15
Thr Cys Leu Val Asn Ser Ser Gln Tyr Ser Arg Trp Asp Phe Phe Thr
            20                  25                  30
Phe Thr Leu Pro Gly Ser Thr Leu Pro Met Arg Lys Glu Phe Thr Ser
        35                  40                  45
Ser Cys Lys Gly Ser Phe Ser Asn His Pro Val Pro Asp His Pro Cys
    50                  55                  60
Lys Leu Val Val Phe Val Arg Pro Lys Met Val Val Thr Ala Gly Ala
65                  70                  75                  80
Asp Ala Tyr Leu Lys Ile Leu Glu Tyr Gly Val Lys Lys Thr Gln Leu
                85                  90                  95
Arg Ile Asp Arg Leu Leu Leu Gln Ala Phe Met Ala Gly Ile Phe Val
            100                 105                 110
Ala Met Ala Gly His Cys Cys Thr Val Leu Ala Gly Ser Tyr Pro Thr
        115                 120                 125
Asp Pro Gly Asp Pro Leu Ala Val Ala Lys Pro Thr Gln Lys Phe Ile
    130                 135                 140
Tyr Gly Ala Leu Phe Pro Val Ala Phe Ile Cys Ile Ile Leu Thr Gly
145                 150                 155                 160
Ala Glu Leu Phe Thr Gly Asn Thr Met Thr Met Leu Ile Cys Tyr Phe
                165                 170                 175
Gln Lys Arg Val Thr Met Leu Gln Leu Gly Val Asn Trp Leu Gly Ser
            180                 185                 190
Leu Ala Gly Asn Trp Leu Gly Ala Leu Phe Gly Ala Tyr Phe Leu Ser
        195                 200                 205
Tyr Leu Thr Gly Ala Leu Gly Asp Glu His Val Arg Gln Phe Leu Phe
    210                 215                 220
Arg Thr Cys Val Asn Lys Ile Ser Tyr Gly Trp Gly Glu Cys Phe Leu
225                 230                 235                 240
Arg Gly Val Gly Cys Asn Thr Phe Val Cys Leu Ala Val Trp Ala Val
                245                 250                 255
Ile Ala Ser Glu Asn Val Ala Gly Lys Val Leu Val Met Trp Phe Pro
            260                 265                 270
Ile Val Ala Phe Cys Val Gly Gly Tyr Glu His Ile Ile Ala Asn Met
        275                 280                 285
Tyr Thr Leu Gln Ala Gly Leu Met Ala Gly Ala Pro Val Ala Ile Leu
    290                 295                 300
Asp Val Ile Ala Phe Asn Phe Leu Pro Thr Leu Leu Gly Asn Ile Val
305                 310                 315                 320
Gly Gly Cys Leu Leu Val Gly Ala Val Tyr Ala Tyr Asn Phe Tyr Pro
                325                 330                 335
Thr Leu Ser Tyr Thr Glu Thr Thr Gly Ala Lys Val Tyr Val Gln Glu
            340                 345                 350
Val Gly Pro Val Leu Asp Arg Arg Ser Ser Met Gln Val Ser Met Thr
        355                 360                 365
Glu Arg Glu Pro Asp Gly Gln Val Val Thr Glu Tyr Glu Ala Val Pro
    370                 375                 380
Phe Glu Ser Phe Gly Gly Glu Tyr Ile Val Asn Lys His Ala Thr Met
385                 390                 395                 400
Ala Ala Pro Ile Pro Ser Arg Ala Ser Ser Phe Leu Tyr Pro Phe Gln
                405                 410                 415
Trp Gln Arg Gln Arg Ser Gln Ser Gly Asn Leu Ser Thr His Ala Arg
            420                 425                 430
Leu Asp Leu Pro Asn Arg Pro Val Glu Pro Pro Ser Asp Gly Leu Glu
        435                 440                 445

Val Thr Pro Gln Ser Gln Thr Ala Glu Ser Val Ala Gln Gln Val
    450                 455                 460

<210> 4
<211> 501
<212> PRT
<213> Toxoplasma gondii

<220>
<223> TgFNT2_TGGT1_229170_ToxoDB

<400> 4
Met Val Leu Ala Ala Ser Pro Glu Ala Tyr Arg Lys Val Ile Glu Tyr
1               5                   10                  15
Gly Ile Lys Lys Thr Lys Leu Arg Ile Asp Arg Leu Phe Leu Gln Ala
            20                  25                  30
Ile Met Ala Gly Ile Tyr Val Gly Met Ala Gly His Ala Cys Thr Ala
        35                  40                  45
Leu Ala Gly Ala Tyr Ser Thr Asp Pro Ala Asn Pro Leu Ala Val Ser
    50                  55                  60
Lys Ala Thr Gln Lys Phe Leu Tyr Ala Ser Leu Phe Pro Val Ala Phe
65                  70                  75                  80
Ile Ala Ile Ile Phe Thr Gly Ala Glu Leu Phe Thr Gly Asn Thr Met
                85                  90                  95
Thr Met Leu Val Cys Leu Leu Glu Arg Arg Val Thr Ala Leu Gln Leu
            100                 105                 110
Cys Ile Asn Trp Ile Cys Ser Leu Val Gly Asn Trp Ala Gly Ala Leu
        115                 120                 125
Phe Ala Ala Tyr Phe Leu Ser Tyr Leu Pro Gly Val Leu Gln Asp Pro
    130                 135                 140
Asp His Leu His Tyr Leu Glu Asp Val Ala Ala His Lys Thr Glu Leu
145                 150                 155                 160
Ser Phe Leu Gln Cys Phe Cys Leu Ala Val Gly Cys Asn Thr Phe Val
                165                 170                 175
Cys Leu Ala Val Trp Phe Val Ile Ala Ser Asp Asp Ala Ala Gly Lys
            180                 185                 190
Ile Met Ser Met Trp Phe Pro Ile Val Ser Phe Cys Val Ala Gly Tyr
        195                 200                 205
Glu His Ile Ile Ala Asn Phe Tyr Thr Leu Gln Cys Ala Leu Met His
    210                 215                 220
Gly Val Gly Pro Gly Val Gly Thr Val Ile Leu Lys Asn Phe Ile Pro
225                 230                 235                 240
Thr Leu Leu Gly Asn Ile Val Gly Gly Cys Gly Leu Val Gly Ala Val
                245                 250                 255
Tyr Trp Tyr Asn Phe Tyr Pro Thr Val Cys Val Val Gln Glu Ala Arg
            260                 265                 270
Gln Pro Leu Pro Leu Ser Glu Asn Ala Pro Ser Ser Thr Arg Gln Val
        275                 280                 285
Val Ala Asp Leu Phe Ser Leu Trp Gly Arg Glu Ser Ser Thr Pro Gly
    290                 295                 300
Val Ser Ala Ser Pro Pro Asp Ala Ala Thr Asn Ala Gly Cys Ser Ala
305                 310                 315                 320
Leu Asp Pro Pro Arg Asn Ala Leu Leu Ala Ala Gly Lys Asn Phe Gly
                325                 330                 335
Asn Leu Ser Ala Gly Asp Arg Gly Ala Leu Ala Glu Gly Ile Pro Gly
            340                 345                 350
Gly Ala Cys Glu Asp Cys Leu Leu Val Pro Arg Ala Ser Phe Gly Gly
        355                 360                 365
Glu Tyr His Pro Pro Gln Gln Gly Asp Ala Gly Arg Trp Cys Lys Pro
    370                 375                 380
Ser Lys Ala Ala Val Gly Ser Gly Gly Val Leu Cys His Val Gln Ser
385                 390                 395                 400
Pro Ala Ala Leu Glu Ala Val Ser Asn Ser Pro Leu Arg Glu Asn Ser

                    405                     410                     415
Gly Val Pro Ser Gly Gly Leu Leu Leu Cys Glu Gly Arg Val Arg Arg
                420                     425                     430
Ser Ser Arg Glu Arg Glu Pro Glu Arg Gly Gly Glu Glu Glu Glu Gly
            435                     440                     445
Ala Ser Pro Glu Glu Glu His Pro Ala Val Thr Leu Ser Ile Pro Pro
    450                     455                     460
Thr Asp Phe His Pro His Val Pro Arg Glu Val Glu Gln Ser Ser Leu
465                     470                     475                     480
Leu Glu Glu Thr Arg Val Ala Ala Glu Asn Ser Ala Leu Glu Glu His
                485                     490                     495
Pro Ala Ser Thr Ile
            500

<210> 5
<211> 356
<212> PRT
<213> Entamoeba histolytica

<220>
<223> EhFNT_EHI_198990_AmoebaDB

<400> 5
Met Pro Arg Glu Lys Pro Arg Ala Asp Glu Ile Ala Ile Glu Met Met
1               5                   10                  15
Ser Val Cys Glu Asp Glu Thr Glu Val Glu Gln Asp Pro Arg Glu Leu
            20                  25                  30
Tyr Glu Glu Glu Met Lys Glu Gln Gln Gln Ile Asp Cys Ser Lys Gln
        35                  40                  45
Gln Lys Glu Val Val Ala Ile Glu Glu Leu Glu Lys Arg Asn Ile Asn
    50                  55                  60
Lys His Phe Phe Ser Ile Gln Pro Asn Thr Gln Ile Pro Val Ile Ser
65                  70                  75                  80
Ser Asn Tyr Ile Ala Pro Val Asp Thr Ser Arg Leu Leu Val Leu Ile
                85                  90                  95
Gly Lys Thr Lys Ala Thr Tyr Pro Ile Met Lys Met Phe Ser Leu Ser
            100                 105                 110
Val Leu Ala Gly Met Leu Leu Ser Val Gly Gly Leu Leu Ser Ile Thr
        115                 120                 125
Ile Gly Lys Gly Ile Pro Ser Ser Asp Ile Gly Ile Gln Lys Ile Val
    130                 135                 140
Phe Gly Phe Phe Asn Ser Val Gly Leu Asn Leu Val Val Leu Cys Gly
145                 150                 155                 160
Gly Glu Leu Phe Thr Ser Asn Cys Ala Phe Leu Ile Pro Gly Phe Met
                165                 170                 175
Glu Gly Ala Tyr Ser Arg Trp Leu Phe Phe Lys Thr His Phe Val Val
            180                 185                 190
Tyr Phe Gly Asn Leu Val Gly Ser Ile Phe Val Ser Thr Tyr Phe Gly
        195                 200                 205
Lys Leu Leu Gly Ser Phe Glu Ser Pro Met Tyr Leu Ser Ala Val Lys
    210                 215                 220
Gln Ile Gly Glu Thr Lys Val Ala Met Asn Trp Gly Arg Ala Leu Leu
225                 230                 235                 240
Ser Gly Ile Gly Cys Asn Trp Leu Val Cys Cys Ala Val Tyr Phe Ser
                245                 250                 255
Ala Ser Ala Lys Asp Leu Leu Ser Lys Leu Val Val Ile Ser Phe Leu
            260                 265                 270
Val Leu Thr Phe Ala Ser Leu Glu Phe Glu Asn Cys Val Gly Asn Met
        275                 280                 285
Phe Leu Leu Ser Leu Ser His Met Tyr Gly Gly Asn Phe Thr Leu Gly
    290                 295                 300
Gln Trp Ile Leu Asn Asn Leu Ile Pro Val Ser Ile Gly Asn Phe Ile
305                 310                 315                 320

Gly Gly Thr Phe Leu Leu Gly Ile Pro Leu Trp Tyr Val His Val Ser
                325                 330                 335
Asn Val Tyr Asn Ile Pro Phe Leu Asp Pro Leu Tyr Gln Gln Ser Gln
            340                 345                 350
Ala Lys Thr Gln
        355

<210> 6
<211> 269
<212> PRT
<213> Salmonella enterica subsp. enterica serovar Typhimurium

<220>
<223> StNirC_AAA27040_NCBI

<400> 6
Met Phe Thr Asp Ser Ile Asn Lys Cys Ala Ala Lys Leu Arg Ala Ser
1               5                   10                  15
Ala Pro Val Ser Ala Asn Asn Pro Leu Gly Phe Trp Val Ser Ser Ala
            20                  25                  30
Met Ala Gly Ala Tyr Val Gly Leu Gly Ile Ile Leu Ile Phe Thr Leu
        35                  40                  45
Gly Asn Leu Leu Asp Pro Ser Val Arg Pro Leu Val Met Gly Ala Thr
    50                  55                  60
Phe Gly Ile Ala Leu Thr Leu Val Ile Ile Ala Gly Ser Glu Leu Phe
65                  70                  75                  80
Thr Gly His Thr Met Phe Leu Thr Leu Gly Val Lys Ala Gly Thr Ile
                85                  90                  95
Ser His Gly Gln Met Trp Ala Ile Leu Pro Gln Thr Trp Leu Gly Asn
            100                 105                 110
Leu Val Gly Ser Val Phe Val Ala Leu Leu Tyr Ser Trp Gly Gly Gly
        115                 120                 125
Ser Leu Leu Pro Val Asp Thr Ser Ile Val His Ser Val Ala Leu Ala
    130                 135                 140
Lys Thr Thr Ala Pro Ala Thr Val Leu Phe Phe Lys Gly Ala Leu Cys
145                 150                 155                 160
Asn Trp Leu Val Cys Leu Ala Ile Trp Met Ala Ile Arg Thr Glu Gly
                165                 170                 175
Thr Ala Lys Phe Leu Ala Ile Trp Trp Cys Leu Leu Ala Phe Ile Ala
            180                 185                 190
Ser Gly Tyr Glu His Ser Val Ala Asn Met Thr Leu Phe Ala Leu Ser
        195                 200                 205
Trp Phe Gly His His Ser Asp Ala Tyr Thr Leu Ala Gly Ile Gly His
    210                 215                 220
Asn Leu Leu Trp Val Thr Leu Gly Asn Thr Leu Ser Gly Val Val Phe
225                 230                 235                 240
Met Gly Leu Gly Tyr Trp Tyr Ala Thr Pro Lys Ser Glu Arg Pro Ala
                245                 250                 255
Pro Ala Lys Ile Asn Gln Pro Glu Ala Ala Ala Asn Asn
            260                 265